# EUROPÄISCHE PATENTSCHRIFT

(11) **EP 0 454 646 B1**
(45) Veröffentlichungstag und Bekanntmachung des Hinweises auf die Patenterteilung: **01.12.1993**
(21) Anmeldenummer: 91850103.2
(22) Anmeldetag: 23.04.1991
(51) Int. Cl.: A61F 2/42

(54) **System für die Rekonstruktion von Gelenken, besonders Handgelenken**
Joint reconstruction system especially for wrist joints
Système de reconstruction pour des articulations notamment pour articulations de poignet

(30) Priorität: 26.04.1990 SE 9001521
(43) Veröffentlichungstag der Anmeldung: 30.10.1991
(73) Patentinhaber: MEDEVELOP AB, S-431 69 Mölndal (SE)
(72) Erfinder: Bränemark, Per-Ingvar, S-431 69 Mölndal (SE)
(74) Vertreter: Barnieske, Hans Wolfgang

(56) Entgegenhaltungen:
- EP-A- 0 396 519
- WO-A-89/03663
- DE-A- 2 309 432
- DE-A- 2 527 864
- DE-A- 2 726 379

## Beschreibung

Die Erfindung bezieht sich auf ein System zur Rekonstruktion von Gelenken, beispielsweise Handgelenken. Ein system gemäß den Merkmalen des Oberbegriffs des Anspruchs 1 ist aus der WO-A-89 03 663 bekannt.

Bisher sind nur wenige Rekonstruktionssysteme für Handgelenke bekannt. Der Ausdruck Rekonstruktion bedeutet in diesem Zusammenhang einen Austausch normaler, in irgendeinerweise beschädigter Gelenke durch künstliche Gelenke. Meistens wird in diesem Zusammenhang die s.g. Swanson Prothese verwendet - diese besteht aus einem aus elastomerem Material mit mittigem Gelenkkörper und an gegenüberliegenden Seiten ausgebildeten, sich stiftförmig verjüngenden Verlängerungen. Nach operativer Entfernung des normalen, beschädigten Gelenkes werden Knochenmarkhöhlungen in den beidseitig vom Gelenk freigelegten Knochenteilen eingebohrt und der künstliche Gelenkkörper mit seinen jeweiligen stiftförmigen Verlängerungen in diese Knochenmarkhöhlungen eingesetzt.

Die unablässige Friktion zwischen diesen stiftförmigen Verlängerungen und dem Knochenmark bzw. dem angrenzenden Gewebe ergibt nach einiger Zeit erhebliche Gewebebeschädungen mit folgenden Inflammationsrisiken und ausserdem werden natürlich die Knochenmarkaushöhlungen durch das ständige Hin- und Hergleiten dieser stiftförmigen Verlängerungen unzulässig und unkontrollierbar erweitert und natürlich auch beschädigt. Praktische Versuche haben ergeben, dass derartige bisher verwendete künstliche Gelenkersatzvorrichtungen schon nach verhältnissmässig kurzer Zeit wieder ausgetauscht werden müssen und dieser zweite Austausch ist schwierig, da man dann mit schon beschädigten Knochenmarkhöhlungen arbeiten muss. Hierüberhinaus hat sich erwiesen, dass das verwendete elastomere Material keine dauerhafte Beanspruchung verträgt. Der grösste Nachteil mit diesem bekannten künstlichen Gelenkersatz ist jedoch darin zu sehen, dass man hiermit das vorliegende Problem nur teilweise lösen kann, da dieser künstliche Gelenkersatz möglicherweise während einer beschränkten Zeit ein "normales" Abbiegen des Handgelenkes zulässt jedoch keine seitliche Drehung des Handgelenkes ermöglicht.

Zweck vorliegende Erfindung ist nun die Schaffung eines künstlichen Gelenkersatzes, besonders für Handgelenke, der sowohl die "normale Abbiegung" des Handgelenkes sowie auch die notwendige seitliche Drehung des Handgelenkes zulässt und somit sämtliche Funktionen eines normalen, unbeschädigten Gelenkes verwirklicht und im übrigen natürlich auch die anderen oben angegebenen Nachteile eliminiert.

Die wesentlichen Kennzeichen des erfindungsgemässen Systemes gehen aus den beiliegenden Ansprüchen hervor.

Die Erfindung wird nachstehend unter Hinweis auf eine in den beigefügten Zeichnungen gezeigte Ausführungsform näher beschrieben, wobei
Fig. 1 den strukturellen Aufbau einer Hand mit einem normalen, unbeschädigten Handgelenk zeigt - von der Innenseite gesehen -,
Fig. 2 in perspektivischer Ansicht die verschiedenen Komponenten des erfindungsgemäss vorgeschlagenden Systemes zur Rekonstruktion eines Handgelenkes darstellt,
Fig. 3 und 4 in verschiedenen Ansichten die im erfindungsgemässen System verwendete erste Hauptkomponente veranschaulicht,
Fig. 5 und 6 in verschiedenen Ansichten die im erfindungsgemässen System verwendete zweite Hauptkomponente veranschaulicht und
Fig. 7 eine Ansicht des erfindungsgemäss applizierten Systemes darstellt.

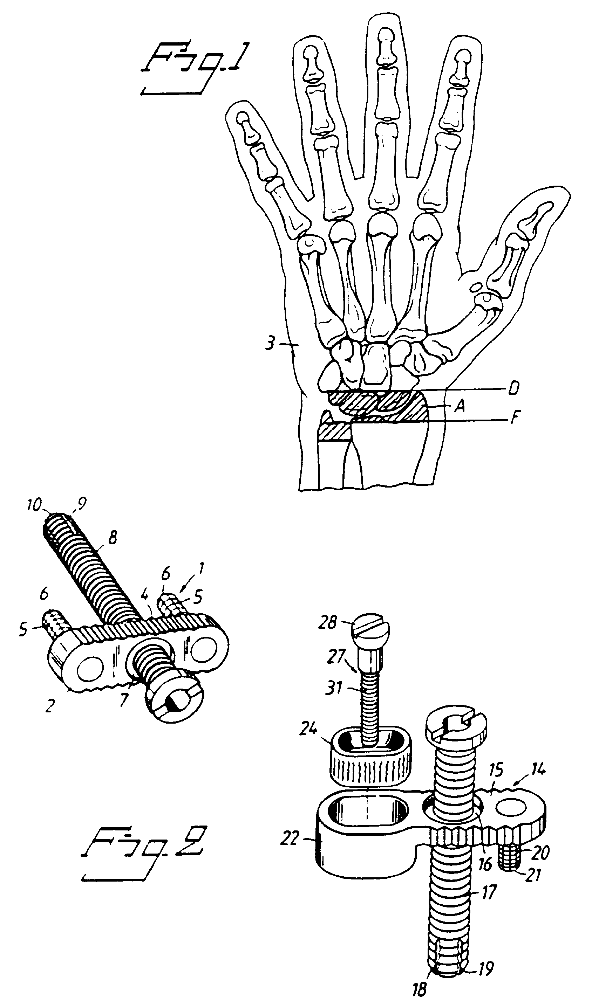

Fig. 1 zeigt den Aufbau eines normalen, unbeschädigten Handgelenkes. Zwecks Rekonstruktion eines beschädigten Handgelenkes durch Verwendung des erfindungsgemäss vorgeschlagenen Systemes werden zunächst die in Fig. 1 gestrichelten, mit A bezeichneten Teile operativ entfernt und mit einem erfindungsgemässen künstlichen Gelenk wie nachstehend beschrieben ersetzt.

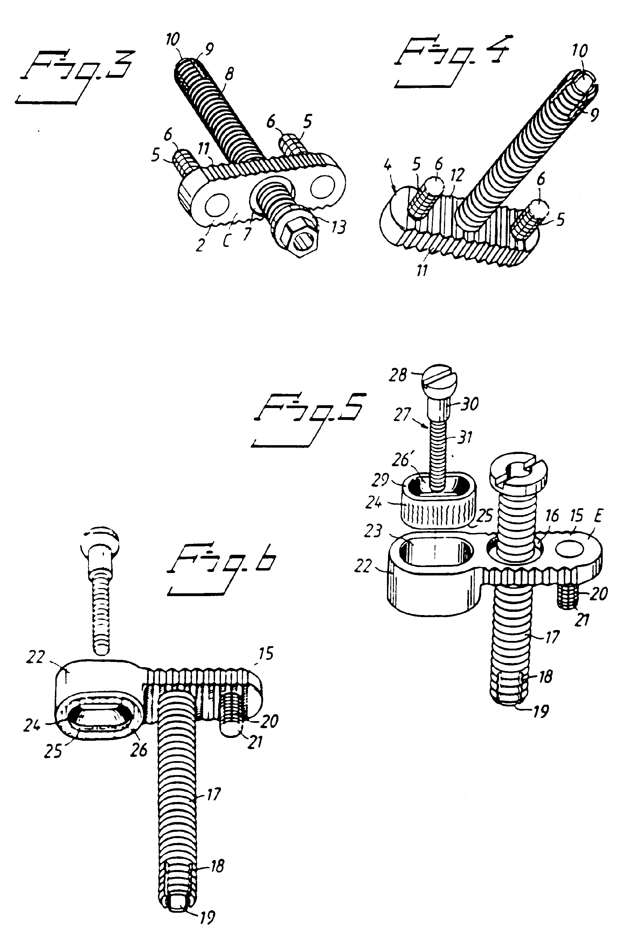

Der prinzipielle Aufbau des erfindungsgemäss vorgeschlagenen Systemes geht aus Fig. 2 hervor, wo eine erste Hauptkomponente mit 1 bezeichnet ist. Diese erste Hauptkomponente besteht aus einer Frontplatte 2 die in ihrer, in applizierter Lage gegen die Handfläche 3 gerichteten oberen Seite zwei im Abstand voneinander angeordnete und in Richtung zur Handfläche 3 herausragende, wenigstens teilweise als Hohlzylinder ausbildete Zapfen 5 mit geschlossenen Enden 6 aufweist. Zwischen den Zapfen 5 ist eine Ausnehmung 7 zur Aufnahme eines schraubenförmigen Verankerungsorganes 8 vorgesehen, und dieses Verankerungsorgan 8 ist in an sich bekannter Weise - vgl hierzu beispielsweise SE A 89 01 508-5 - teilweise als Hohlkörper ausgebildet und an seinem offenen Ende 10 mit schlitzförmigen Einschnitten 9 versehen, die vorzugsweise mit Schneidkanten ausgebildet sind.

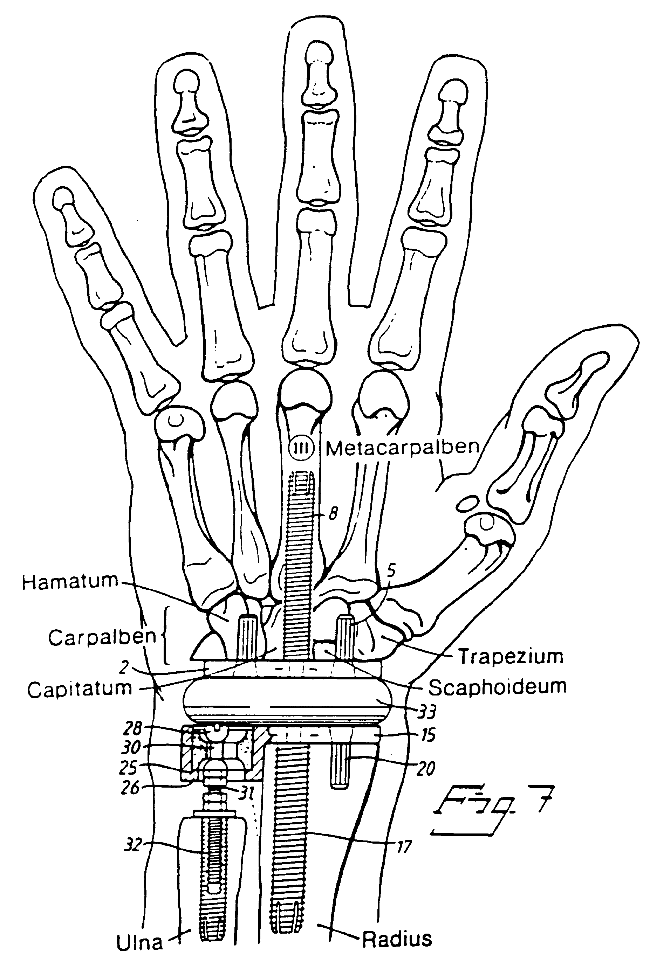

Weitere Einzelheiten der ersten Hauptkomponente des erfindungsgemäss vorgeschlagenen Systemes gehen aus Fig. 3 und 4 hervor.

Diese erste Hauptkomponente ist gänzlich aus einem biokompatiblem d.h. gewebeverträglichem Material hergestellt oder wenigstens mit einem äusseren Belag eines biokompatiblen Materiales versehen, beispielsweise Titan. Die Frontplatte 2 ist zweckmässig an ihren Seitenkanten mit Einkerbungen 11 oder dergleichen versehen, die in die Einführungsrichtung der Frontplatte verlaufen und zu einem verbesserten Einwachsen der Frontplatte vorgesehen sind. Zweckmässiger Weise können die Oberflächen der ersten Hauptkomponente falls diese aus Titan bestehen oder mit Titan beschichtet sind, Mikrodellen von etwa 10 - 1000 nm aufweisen, wodurch in bekannter Weise ein ausserordentliches Einwachsen des Implantates im Gewebe erreicht wird, d.h. durch sogennante Osseointegration, wobei die Ausläufer der Gewebezellen mit den oben genannten, dellenförmigen Vertiefungen in der Oberfläche zusammenwirken. Auch die Oberseite 4 der Frontplatte 2 ist geriffelt - Bezugszeichen 12. Die Öffnung 7 in der Frontplatte 2 ist etwas versenkt zwecks Aufnahme des oberen Flansches 13 des Verankerungsorganes 8.

Die zweite Hauptkomponente des erfindungsgemäss vorgeschlagenes Systemes wird generell mit 14 bezeichnet und ist näher in den Figuren 2, 5, 6 und 7 veranschaulicht.

Auch die zweite Hauptkomponente hat eine Frontplatte 15 mit einer Öffnung 16 zur Aufnahme eines schraubenförmigen Verankerungsorganes 17, das in gleicher Weise wie das Verankerungselement 8 der ersten Hauptkomponente 1 wenigstens teilweise hohl und mit von seinem unteren Ende 19 ausgehenden Einschlitzungen 18 ausgebildet ist, die zweckmässig als Schneidkanten ausgeführt sind.

Auf der einen Seite der Öffnung 16 ist ein Hohlzapfen 20 mit geschlossenem Ende 21 angeordnet und auf der entgegenge setzten Seite der Öffnung 16 geht die Frontplatte 15 in einen hülsenförmigen, in der gleichen Richtung wie der Zapfen 20 von der Frontplatte 15 hervorragenden Steuerkörper 22 über. Der Steuerkörper 22 ist mit einer hauptsächlich ovalen Bohrung 23 versehen, die einen hauptsächlich ovalen Einsatz 24 aufnimmt, der mit seiner unteren Begrenzung 25 wenigstens teilweise an einem nach innen gerichteten Endflansch 26 des Steuerkörper 22 anliegt. Der Einsatz 24 ist mit einem schlitzförmigen länglichen Steuerspalt 26' versehen und zur Aufnahme eines Steuerrorganes 27 mit einem Steuerkopf 28, der zum Anliegen gegen die obere Begrenzung 29 des Schlitzes vorgesehen ist, bestimmt, sodass die an den Kopf 28 des Steuerorganes 27 anschliessende glatte Partie 30 prinzipiell im Einsatz 24 angeordnet ist.

Das Steuerorgan 27 ist hierüberhinaus mit einem unteren Gewindeteil 31 versehen, und dient zum Festschrauben in einer im aktuellen Beingewebe (Ulna) angeordneten Fixtur 32.

Sämtliche Teile der zweiten Hauptkomponente - ausser dem Einsatz 24 - sind aus einem biokompatiblen Material, vorzugsweise Titan, hergestellt, und auch vorzugsweise mit den oben genannten Oberflächendellen versehen. Der Einsatz 24 dahingegen ist aus einem zweckmässigen Kunststoffmaterial, beispielsweise Polyäthylen hergestellt.

Das erfindungsgemäss vorgeschlagene System umfasst ausserdem eine dritte Hauptkomponente 33 in Form einer Gelenkmechanik, die beispielsweise aus einem elastischen Kissen aus Silikonkunststoff bestehen kann. Dieses Kissen wird zwischen den Frontplatten 2 bzw 15 der ersten und zweiten Hauptkomponente 1 bzw 14 angebracht und wird durch vom Kissen 33 herausragende, zapfenförmige Organe lagefixiert, wobei diese in die Hohlzapfen 5 der Frontplatte 2 und in die Hohlzapfen 20 bzw in den oberen hohlen Teil der Schraube 17 der zweiten Hauptkomponente eingeführt werden.

Des erfindungsgemäss vorgeschlagenen System wird in folgender Weise verwendet.

Zunächst werden die defekten, natürlichen Teile des Handgelenkes, d.h. die gestricherten Partien A nach Fig. 1, durch operativen Eingriff entfernt. Danach wird in an sich bekannterweise eine Titanfixtur in die Ulna zwecks späterer Aufnahme des Gewindes 31 des Steuerorganes 27 angebracht.

Hiernach wird die Frontplatte 2 der ersten Hauptkomponente nach notwendigen Präparationen appliziert, so dass die hauptsächlich ebene Frontfläche C der Platte 2 in dergleichen Ebene wie die Linie D (Fig. 1) liegt.

Die Frontplatte 1 wird nun i ihrer Lage fixiert wobei die Befestigungsschraube 8 in das Metacarpalbein III (Fig. 7) eingeschraubt wird.

Nach notwendigen Präparationen wird danach die Frontplatte 15 der zweiten Hauptkomponente 14 so angebracht, dass die ebene Seite E der Frontplatte 15 hauptsächlich in der gleichen Ebene wie die Linie F in Fig. 1 liegt. Die Frontplatte 15 wird danach in ihrer Lage fixiert durch Einschrauben der Befestigungsschraube 17 im Radius. Danach wird der Einsatz 24 im Steuerkörper 22 eingelegt, wobei das Steuerorgan 27, von oben durch den Einsatz 24 geführt wird und mit seinem unteren Gewindeteil 31 in der Fixtur 32 fixiert wird. Hierbei kann die Einschraublänge des Gewindeteiles 31 mittels Stopporgane in Form von Muttern oder dergleichen variiert werden. Schliesslich wird der Gelenkmechanismus 33 zwischen den Frontplatten angeordnet und in oben angebener Weise fixiert.

Dieses so applizierte System ergibt eine Gelenkfunktion die mit einem natürlichen Gelenk gleichstellbar ist. Die hauptsächliche Abbiegung des Handgelenkes wird durch den Gelenkmechanismus 33 erreicht und ausserdem wird die angestrebte Rotation des Handgelenkes (d.h. die Rotation des Radius um die Ulna) durch das vorgeschlagene, neuartige Anordnen des Steueroragenes 27 erreicht, wodurch eine Rotationsbewegung von +/- 90° ermöglicht wird.

Die Erfindung ist natürlich nicht auf die in den beigefügten Zeichnungen gezeigten Ausführungsformen beschränkt sondern kann in verschiedenster Weise variiert werden.

Im übrigen kann das erfindungsgemäss vorgeschlagene System mit kleinen Modifikationen auch als Ersatz eines natürlichen, beschädigten Armgelenkes verwendet werden.

## Patentansprüche

1. System zur Rekonstruktion von Gelenken, insbesondere Handgelenken, welches System
eine erste Hauptkomponente (1) mit einer ersten Frontplatte (2),
eine zweite Hauptkomponente (14) mit einer zweiten Frontplatte (15) sowie
eine dritte Hauptkomponente (33) aufweist, wobei die dritte Hauptkomponente (33) aus einem künstlichen Gelenkmechanismus aus elastischem Material besteht und zwischen den beiden gegeneinander gerichteten Flächen der bezüglichen Frontplatten (2, 15) lagefixiert ist, um durch Deformation des elastischen Materiales eine Gelenkabbiegung zu verwirklichen, dadurch gekennzeichnet, daß die erste Frontplatte (2) ein in das gelenknahe Knochengewebe einschraubbares Verankerungsorgan (8) aufnimmt sowie wenigstens einen, von der ersten Frontplatte (2) in Einschraubrichtung des Verankerungsorganes (8) herausragenden, teilweise hohl ausgebildeten Zapfen (5) aufweist, und daß die zweite Frontplatte (15) ein in das entgegengesetzte, gelenknahe Knochengewebe einschraubbares Verankerungselement (17) aufnimmt und wenigstens einem in Einschraubrichtung des Verankerungselementes (17) von der zweiten Frontplatte (15) hervorragenden Hohlzapfen (20) aufweist.

2. System nach Anspruch 1, dadurch **gekennzeichnet** dass die dritte Hauptkomponente (33) mittels von dieser herausragenden Stiften in den in den Frontplatten (2 bzw 14) vorhandenen Hohlzapfen (5 bzw 20) arretierbar ist.

3. System nach Anspruch 1, dadurch **gekennzeichnet**, dass die zweite Hauptkomponente (14) einen Steuerkörper (22) aufweist, der mit einem in der Ulna befestigbaren Steuerorgan (27) zwecks Ermöglichung einer seitlichen Drehung des rekonstruierten Gelenkes zusammenwirkt, wobei der Oberteil des Steuerkörpers (22) eine ovale schlitzförmige Steuerungsspalte zur Aufnahme des Oberteiles des Steuerorganes (27) aufweist.

4. System nach Anspruch 3, dadurch **gekennzeichnet**, dass die schlitzförmige, ovale Steuerungsspalte in einem in den Oberteil des Steuerkörpers (22) einsetzbaren Einsatz (24) aus polymerem Material ausgebildet ist.

5. System nach Anspruch 3 oder 4, dadurch **gekennzeichnet,** dass das Steuerorgan einen etwa kugelförmigen kopf (28) zum Zusammenwirken mit dem Steuerspalt aufweist und der Steuerspalt im Verhältnis zum Steuerrorgan (27) so dimensioniert ist, dass eine seitliche Bewegung des Steuerorganes im Steuerschlitz zugelassen ist.

6. System nach den Ansprüchen 3 - 5, dadurch **gekennzeichnet**, dass das Steuerorgan (27) mit seinem unteren Gewindeteil in eine in der Ulna vorhandene Fixtur eingeschraubbar ist, wobei die Einschraublänge durch zweckmässige Stopporgane variiert werden kann.

7. System nach einem oder mehreren der vorhergehenden Ansprüche, dadurch **gekennzeichnet**, dass die verschiedenen Teile des Systemes ausser dem Einsatz und dem Gelenkmechanismus aus Titan hergestellt sind.

## Claims

1. A system for reconstructing joints, more particularly wrists, the system having:
a first component (1) having a first front plate (2);
a second main component (14) having a second front plate (15), and
a third main component (33), the same comprising a prosthetic joint mechanism made of a resilient material and being secured in position between the two facing surfaces of the front plates (2, 15) in order to facilitate bending of the wrist by deformation of the resilient material,
characterised in that
the first front plate (2) receives a fixing member (8) adapted to be screwed into the bone tissue near the joint and at least one partly hollow pin (5) which projects from the first front plate (2) in the screwing-in direction of the fixing member (8), and the second front plate (15) receives a fixing element (17) adapted to be screwed into the opposite bone tissue near the joint and has at least one hollow pin (20) which projects from the second front plate (15) in the screwing-in direction of the fixing element (17).

2. A system according to claim 1, characterised in that the third main component (33) can be secured in the hollow pins (5, 20 respectively) in the front plates (2, 14 respectively) by means of pins which project from the component (33).

3. A system according to claim 1, characterised in that the second main component (14) has a guide member (22) which co-operates with a guide member (27) adapted to be secured in the ulna to facilitate lateral rotation of the reconstructed wrist joint, the top part of the guide member 22 being formed with an oval slot-like gap to receive the top part of the guide member 27.

4. A system according to claim 3, characterised in that the slot-like oval gap is present in an insert (24) which is made of polymeric material and which is introducible into the top part of the guide member (22).

5. A system according to claim 3 or 4, characterised in that the guide member has a substantially spherical head (28) for co-operation with the gap and the same is so dimensioned relatively to the guide member (27) that the same has provision for lateral movement in the guide slot.

6. A system according to claims 3 - 5, characterised in that the guide member (27) is screwable by its bottom screwthreaded part into a fixture in the ulna, the screwed-in length being variable by appropriate stop elements.

7. A system according to one or more of the previous claims, characterised in that various parts of the system except for the insert and the joint mechanism are made of titanium.

## Revendications

1. Système de reconstruction pour les articulations, notamment pour articulations du poignet, système qui présente un premier composant principal (1) avec une première plaque avant (2), un deuxième composant principal (14) avec une deuxième plaque avant (15) et un troisième composant principal (33), le troisième composant principal (33) étant composé d'un mécanisme articulé artificiel à base de matériau élastique et étant fixé dans sa position entre les deux surfaces orientées l'une vers l'autre des plaques avant correspondantes (2,15) pour réaliser une flexion de l'articulation par la déformation du matériau élastique, caractérisé en ce que la première plaque avant (2) reçoit un organe d'ancrage (8) vissable dans le tissu osseux proche de l'articulation et présente au moins un téton (5) en partie creux qui dépasse de la première plaque avant (2) dans le sens de vissage de l'organe d'ancrage (8), et en ce que la deuxième plaque avant (15) reçoit un organe d'ancrage (17) vissable dans le tissu osseux opposé et proche de l'articulation et présente au moins un téton (20) creux qui dépasse de la deuxième plaque avant (15) dans le sens de vissage de l'élément d'ancrage (17).

2. Système selon la revendication 1, caractérisé en ce que le troisième composant principal (33) est verrouillable au moyen de broches dépassant de celui-ci dans les tétons creux (5 et 20) se trouvant dans les plaques avant (2 et 15).

3. Système selon la revendication 1, caractérisé en ce que le deuxième composant principal (14) présente un corps de commande (22) qui agit de concert avec un organe de commande (27) fixé dans le cubitus pour permettre une rotation latérale de l'articulation reconstruite, la partie supérieure du corps de commande (22) présentant une perforation de commande en forme ovale en forme de fente pour recevoir la partie supérieure de l'organe de commande (27).

4. Système selon la revendication 3, caractérisé en ce que la perforation de commande ovale en forme de fente se trouve dans une pièce d'insertion (24) insérable dans la partie supérieure du corps de commande (22) et à base de matériau polymère.

5. Système selon l'une quelconque des revendications 3 ou 4, caractérisé en ce que l'organe de commande présente une tête sphérique (28) pour l'action conjuguée avec la fente de commande et en ce que la fente de commande est dimensionnée par rapport à l'organe de commande (27) de façon à permettre un mouvement latéral de l'organe de commande dans la fente de commande.

6. Système selon les revendications 3-5, caractérisé en ce que l'organe de commande (27) est vissable avec sa partie filetée inférieure dans un dispositif fixe se trouvant dans le cubitus, la profondeur de vissage pouvant être modifiée au moyen d'éléments d'arrêt appropriés.

7. Système selon une ou plusieurs revendications précédentes, caractérisé en ce que les différentes parties sont fabriquées en titane, sauf la pièce d'insertion et le mécanisme articulé.
